(19) **Europäisches Patentamt**
**European Patent Office**
**Office européen des brevets**

(11) **EP 2 034 928 B1**

(12) **EUROPEAN PATENT SPECIFICATION**

(45) Date of publication and mention
of the grant of the patent:
**04.01.2012 Bulletin 2012/01**

(51) Int Cl.:
**A61F 2/16** *(2006.01)* **G09B 23/28** *(2006.01)*
**G09B 23/30** *(2006.01)*

(21) Application number: **07724604.9**

(22) Date of filing: **26.04.2007**

(86) International application number:
**PCT/EP2007/003674**

(87) International publication number:
**WO 2007/128423 (15.11.2007 Gazette 2007/46)**

(54) **ASPHERIC INTRAOCULAR LENS AND METHOD FOR DESIGNING SUCH IOL**

ASPHÄRISCHE INTRAOKULARLINSE UND VERFAHREN ZUR KONSTRUKTION DIESER IOL

LENTILLE INTRA-OCULAIRE ASPHERIQUE ET PROCEDE DE CONCEPTION D'UNE TELLE LENTILLE INTRA-OCULAIRE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **05.05.2006 DE 102006021521**

(43) Date of publication of application:
**18.03.2009 Bulletin 2009/12**

(73) Proprietor: **Carl Zeiss Meditec AG**
**07745 Jena (DE)**

(72) Inventors:
• **LESAGE, Cédric**
**17220 Sainte-Soulle (FR)**
• **GERLACH, Mario**
**07768 Altenberga (DE)**

(74) Representative: **Carl Zeiss AG - Patentabteilung**
**Carl-Zeiss-Strasse 22**
**73447 Oberkochen (DE)**

(56) References cited:
**EP-A- 1 424 049          US-A1- 2006 030 938**
**US-B2- 6 609 793**

• **TAKETANI F ET AL: "Influence of intraocular lens tilt and decentration on wavefront aberrations" JOURNAL CATARACT AND REFRACTIVE SURGERY, AMERICAN SOCIETY OF CATARACT AND REFRACTIVE, US, vol. 30, no. 10, October 2004 (2004-10), pages 2158-2162, XP004595315 ISSN: 0886-3350 cited in the application**
• **KASPER ET AL: "Intraindividual comparison of higher-order aberrations after implantation of aspherical and spherical intraocular lenses as a function of pupil diameter" JOURNAL CATARACT AND REFRACTIVE SURGERY, AMERICAN SOCIETY OF CATARACT AND REFRACTIVE, US, vol. 32, no. 1, January 2006 (2006-01), pages 78-84, XP005628846 ISSN: 0886-3350**

**Description**

**1 Field of invention**

The invention discloses a novel Intraocular lens (IOL) and a method for improvement of such lenses in the field of ophthalmology, which comprise surface shape modifications that differ from perfect spherical geometries.

**2 Background of the invention**

The treatment of Cataract, as the worlds most cause for blindness, is a well known process since the ages of the antique Rome (1st and 2nd century AD). Since that ancient time, the complete removal of the opaque human crystalline lens is still the best choice to partially restore visual acuity of the patients. The achieved results are expectedly bad, because of the disregarded refractive contributions of the natural human lens to the visual apparatus, which is not adequately compensated in this situation.

A breakthrough in Cataract surgery appeared to be in 1949 as the English Doctor Harold Ridley successfully implanted the first intraocular lens made of hard PMMA plastics. This lens was capable to compensate for lost optical power of the natural human lens. Since this early time IOLs and surgery techniques were continuously improved. Today Cataract surgery is the far most performed surgery in ophthalmology with more than 2.3 million patients per year in the United States. Approximately another 3 million surgeries add from Europe and Japan.

The performance of the human eye, in terms of an optical system can only be accomplished if the artificial lens is properly positioned and put into focus. By fulfillment of this condition the incoming rays from distant object points form minimum blurred spots at the retina and provide sharp vision. The correct adaptation of an IOL to individual human eye is still difficult and the post operative visual acuity of the patients depends on several factors.

Inaccuracies while measuring the various ocular geometries, inaccuracies during surgery and post surgery effects, such as surgical trauma and wound healing processes limit the achievable visual acuity due to positioning errors of the implanted IOL. Positioning errors with respect to the optical axis will mainly cause defocus, tilt and decentration of the IOL will result in induced Astigmatism and Coma errors. Higher order optical aberrations will appear as well.

By today, different IOL design approaches deal with these issues and try to mitigate the issues with particular emphasis on certain aspects.

A selection of prior art lens designs is described in brief in the next section:

**1. Equi-Convex Lens Design (example: Bausch & Lomb LI61U)**

The Equi-Convex IOL is the most used intraocular lens design in clinical practice. Both surfaces are spherical with equivalent radius of curvature. As a consequence of these designs produce a significant amount of spherical aberration. Due to the strong increase of spherical aberration with pupil diameter the patients will very likely suffer from blur and contrast loss under mesopic / scotopic conditions due to spherical aberration.

**2. Bi-Convex or Plano Convex Lens Design: (example AMO sensar AR40)**

The additional degrees of freedom allow designing a 'best shaped IOL' that provides minimum spherical aberration that is achievable with spherical surfaces. The amount of spherical aberration is significantly reduced as compared with 1. Since the amount of SA is still higher than with the natural human lens, the patients will very likely suffer from blur and contrast loss under mesopic / scotopic conditions due to spherical aberration.

**3. Wavefront optimized IOLs (example: Pharmacia, TECNIS Z9000)**

Patent: US 6,609,793B2
Anterior surface is aspherical. The deviations from the base sphere are expressed as 6th order polynomial expansion. The IOL design bases on averaged wavefront aberrometry data obtained on a large patient cohort. The objective of the aspherization is to compensate for the positive spherical aberration as induced by the normal human cornea. The IOL has to provide a certain amount of negative spherical aberration to bring the entire optical apparatus to zero spherical aberration. As viewed from a theoretical optics perspective this design should provide maximum optical performance at the narrowest possible point spread function. The TECNIS Z9000 provides diffraction limited optical performance on-axis. This holds true even for large pupils of 6mm. Such lens design has also some disadvantages. Due to the intended significant negative spherical aberration of the lens it becomes very sensitive with respect to decentration that is likely to occur during the implantation and after implantation during capsular bag symphysis. The diffraction limited performance of the lens vanishes immediately even if slightly decentered.

**4. The "aberration-free IOL" (Example: Bausch and Lomb, SofPort A0 and Akreos AO)**

Patents: US US2005/203619A1 and WO2004/090611A3
Both surfaces of the IOL are aspherical and the shape is defined by a conic constant. Considering the specific optical conditions behind the cornea, the IOL does not introduce any additional spherical aberration to the optical system. In other words: It is 'transparent' to the amount of incoming aberrations. Systems that do not introduce spherical aberrations do even not introduce coma while decentered. Therefore these lenses can be significantly decentered without loosing contrast than compared to the perfectly centered state. Since the corneal spherical aberration is not affected by the IOL, this amount of spherical is manifest and limits the optical performance on-axis. The "aberration-free IOL" does not comply with the physiological properties of the natural human lens and therefore it may lead to sub-optimal results. This lens can be used for eyes after refractive surgery, eyes with keratoconus or with atypical corneal spherical aberration.

Some other patents exist on the subject of the increasing of the spherical aberrations in order to provide improved depth of field or pseudoaccommodation as:

Patent US 2004/0230299 (November 18th, 2005)
Oscillate surface superimposed on a spherical surface to produce different focuses before and behind the best focus in a way to obtain an increased depth of focus.

Patent WO 2005/046527 (May 26th, 2005)
Multizone monofocal lens. Each zone presents a positive or negative gradient of power from the base power of the lens in a way to produce an extended depth of field.

Patent US 6,126,286 (October 3rd, 2000)
Multizone monofocal lens to produce an improved depth of field.

Patent EP 1402852 (September 29th, 2003)
Monofocal aspherical lens that permits a pseudoaccommodation by providing an improved dept of field (by increasing the amount of spherical aberrations). EP 1424049 discloses a method of designing a multifocal ophtalmic lens capable of reducing abberations of the eye.

**3 Objective of invention**

The invention shall provide means to overcome the disadvantages of the prior art and shall provide significantly improved perceivable optical performance to patients who need an IOL implant.

**4 Summary of the invention**

The invention provides a new aspheric intraocular lens and a method for designing such IOL that results in obtaining a intraocular lens which provides significantly improved perceivable optical performance to IOL patients.

The aspheric Intraocular lens according to the invention is comprising an anterior and a posterior surface, whereby at least one of the anterior and posterior surface is aspheric and whereby the optical properties of these surfaces account for an spherical aberration equal or nearby the spherical aberration of the human eye.

In another embodiment of the invention the IOL can be made of a material that has a varying refractive index that accounts for an spherical aberration equal or nearby the spherical aberration of the human eye.

The invention also comprises a new method for designing an intraocular lens capable of adjusting the aberrations of the eye in order to provide optimal vision correction to patients consisting of the following elements:

- A mathematical model eye that describes the optical setup and performance of the natural human eye, comprising at least one aspherical corneal surface, a gradient index and / or aspherical crystalline lens model, a visual axis that is tilted with respect to the axis of symmetry 'optical axis' of the eye and a decentered Iris that represents a decentered entrance pupil.
- Determination of the performances of the mathematical model eye in terms of image quality and spherical aberrations in function of the pupil diameter.
- Using a mathematical model that describes the statistics of potential lens misalignments and positioning errors induced by surgery or wound healing processes
- Calculating the optical performance and resulting aberrations employing said mathematical eye model convoluted with the statistical model for lens displacements
- And optical modeling of an aspherical lens shape that replaces the natural human crystalline in the eye that provides optical power restoration while providing having aspherical shape optical characteristics of the human lens in order to make the eye with the intraocular lens to have the same amount of spherical aberrations and the same level of image quality than the mathematical model eye in function of the pupil diameter.

In such a method either one of the anterior or posterior ar both surfaces of the lens may be of aspherical shape.

It is advantageous that the radial distribution of refractive optical power is divided in at least three functional zones that account for photopic, mesopic, and scotopic vision.

Preferably the optical optimization of the aspherical shape is performed in order to minimize sensitivity of the optical performance parameters with respect to potential lens tilt induced by surgery effects or capsular bag symphysis.

Advantageously the optical optimization of the aspherical shape is performed in order to minimize sensitivity of the optical performance parameters with respect to potential lens decentration induced by surgery effects or wound healing processes.

A preferred way of modeling and optimization of the lens shape includes selecting the radii of base curvature of the anterior and posterior surfaces as well as the central thickness, the edge thickness and the refractive index.

In the method according to the invention the amount of spherical aberration of the artificial lens is maintained at the level as the one of the natural human lens over a broad range of pupil diameters.

Preferably the modified lens shape is defined in terms of linear combination of polynomials

The modified lens shape can be defined by the equation:

$$z = \frac{cr^2}{1+\sqrt{1-(1+Q)c^2r^2}} + k_2 r^2 + k_4 r^4 + k_6 r^6 + k_8 r^8$$

with

$$c = r_{curv}^{-1}$$ (Curvature = 1 / base radius of curvature)

r = independent variable, radius about optical axis

Q = conic constant

$k_n$ = polynomial coefficient of order n

Thereby the constant Q can be 0 or between -1 and 0. The coefficient $k_2$ may be, the coefficients $k_n$ for n > 6 may be equal to 0.

Advantageously the modified lens shape is defined in terms of linear combination of polynomials or by splines or piecewise defined by linear combinations of polynomials.

The optical performance can be defined as MTF contrast or Strehl Ratio or wavefront error or in terms of point spread functions and encircled energy.

The aberrations of the entire optical train of the human eye can be expressed in linear combinations of Zemike or Seidel polynomials or Fourier decomposition of the OPD /wavefront.

An aspheric Intraocular lens according to the invention can be made of soft material, or hydrophilic material such as hydrophilic acrylic polymer, or copolymer or a hydrophobic material such as hydrophobic acrylic or silicon.

Also the aspheric Intraocular lens according to the invention can be made of monobloc material with hard and soft zones such as described in patent EP1003446, or of hard material such as polymethylmethacrylathe also know as PMMA

Besides correction of spherical vision errors the surface modifications according to the invention allow the restoration of the optical properties of the natural human lens, as existing prior to extraction. In addition the intentional balancing of the aforesaid anterior and posterior surface modulations provide minimum sensitivity of the optical performance with regards to mechanical positioning disturbances such as decentration and tilt of the IOL that may be induced due to surgery inaccuracy, surgical trauma or capsular bag symphysis.

This is achieved by intentional adjustment of the optical aberrations in a way to make them similar to the effects of the natural human crystalline lens.

The image formation in natural human eye is accomplished by the conjunction of the ocular media and interfaces. The main contribution in refractive power (~75%) is provided by the Cornea which is the first air / media interface of the human eye. Rays emitted by distant object points enter the cornea almost parallel with respect to the optical axis. The refraction of the Cornea bends the rays towards the optical axis into an converging bundle. This bundle of rays passes the anterior chamber and enters the human crystalline lens. If no crystalline is in place the rays would converge to singular diffraction limited small spot at a distance of the inverse corneal power. The spot size is determined by the diffraction effects at the edges of the entrance pupil and the wavelength.

The optical system of the human eye is not perfect in terms of physics but it has developed over ages and optimized itself. The slight aspherical shape of the Cornea acts in conjunction with the non-linear Snell's Law of refraction and does not allow that all rays emitted from a distant point source converge at a singular spot. It appears that rays from the outer portions of the pupil hit the optical axis at a smaller distance than the axial rays do. This effect is called spherical aberration (in the following abbreviated SA) and is related to a sign. If the pupil edge rays hit the optical axis before the axial rays do, the SA is considered to be 'positive'. Positive spherical lenses show this behavior. If the pupil edge rays hit the optical axis at a more distant point on the optical axis than the axial rays the SA is considered to be 'negative'. This behavior is found on plano- parallel glass plates or negative lenses.

Since the edge rays of the cornea hit the optical axis before the axial rays do it adds positive SA to the optical system. This effect prevents the formation of infinitely sharp macular images. Instead there is plenty of light diffusion in blurred spots. The evolution of the human eye accounts for that by developing a highly complex crystalline lens design. The crystalline lens contributes the missing 25% of refractive power to the optical system in order to adjust the focal length exactly to the available axial length of the human eye. In addition it allows accommodating for different viewing distances by internal adjustment of the refractive lens power. Beyond these obvious facts, the crystalline lens acts as an optical correction means of the human eye that compensates for optical errors as introduced by the Cornea. In terms of avoiding excessive spot blurring induced by corneal positive SA, the crystalline provides a well adjusted amount of negative SA that almost perfectly compensates for the amount induced by the cornea. The optical performance of this joint optical system is significantly better than of its single components. This inherent compensation mechanism is even working for different viewing distances and pupil diameters due to different lighting conditions.

The main objective of human eye evolution was not to optimize the theoretical optical performance of the eye such as currently widely announced in terms of point spread functions or Strehl Ratios. However, the optical apparatus should provide optical performance that perfectly matches the requirements of the cone and rod structure of the retina, their local density functions and color perception properties. Cone and rods mosaic permit only to see images with maximal spatial frequency of 75 cpd, higher spatial frequency can produce aliazing and distort the perceived image as described by Y. K. Nio and al in the article "Spherical and irregular aberrations are important for the optimal performance of the human eye", Ophtal. Physiol. Opt. 2002 22103-112. The optical properties of the visual apparatus, the retinal configuration and the physiological processing of the visual information in the visual cortex determine finally the perceivable visual acuity of the patients.

This teaches the main objectives of a novel intraocular lens. The inventors came to the conclusion, that the IOL according to the invention has to restore both optical power and aberration characteristics of the natural human lens in order to support the neuro-visual optical system for best perceivable visual performance. For explanation see for example P. Artal et al. in the article "Neural compensation for the eye's optical aberrations", Journal of Vision (2004)4, 281-287.

The design of the novel intra ocular lens takes into account the natural optical configuration of the human vision apparatus e.g. visual axis tilt and pupil decentration. In addition the method will account for potential positioning errors caused by implantation and surgery effects.

## 5 Brief description of the drawings

Fig. 1: The Liou - Brennan model eye with pupil decentration and visual axis tilt
Fig. 2: Statistical Distribution of IOL positioning errors
Fig. 3: Spherical aberration vs. Pupil diameter for different IOLs
Fig. 4: Layout of the aspherical IOL according to the invention
Fig. 5: Other layout of the aspherical IOL according to the invention
Fig. 6: Radial optical power and corresponding zones for different IOLs
Fig. 7: Strehl Ratio vs. Pupil diameter for different IOLs
Fig. 8 - 13 Modulation transfer function for different IOLs for different pupil diameters, decentrations and tilt angles

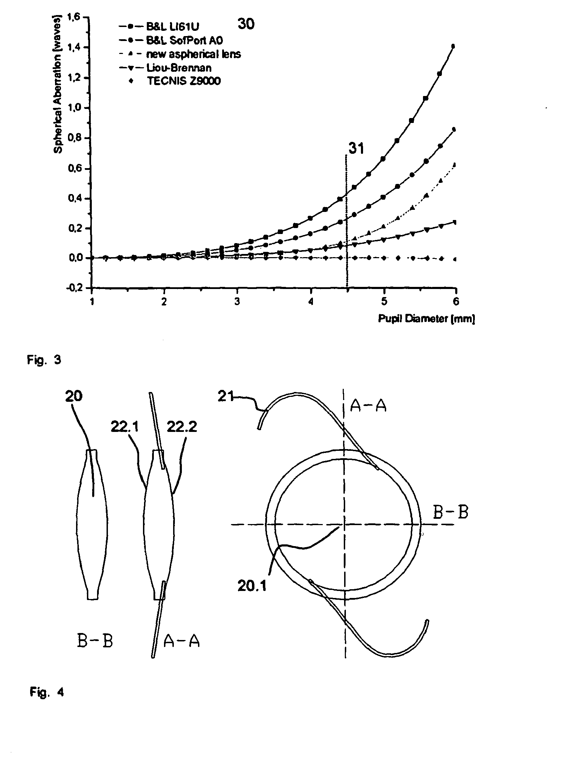

The aspheric IOL design according to the invention is referred as "new aspherical IOL" in Figures 3 and 6 to 13.

## 6 Description of preferred embodiments of the invention

In order to provide a design environment for an IOL a particular theoretical eye model needs to be applied. Many of them are well known from the literature e.g. the *Gullstrand: Helmholtz's Physiological Optics; Norrby et al: "Methods of obtaining ophthalmic lenses providing the eye with reduced aberrations" US patent US* 6, 609, 793*; or Thibos et al*: "*A new single surface eye that accurately predict chromatic and spherical aberrations in the human eye*",

*Invest. Ophtal. Visual Sci. 34*, 777 (*1993*).

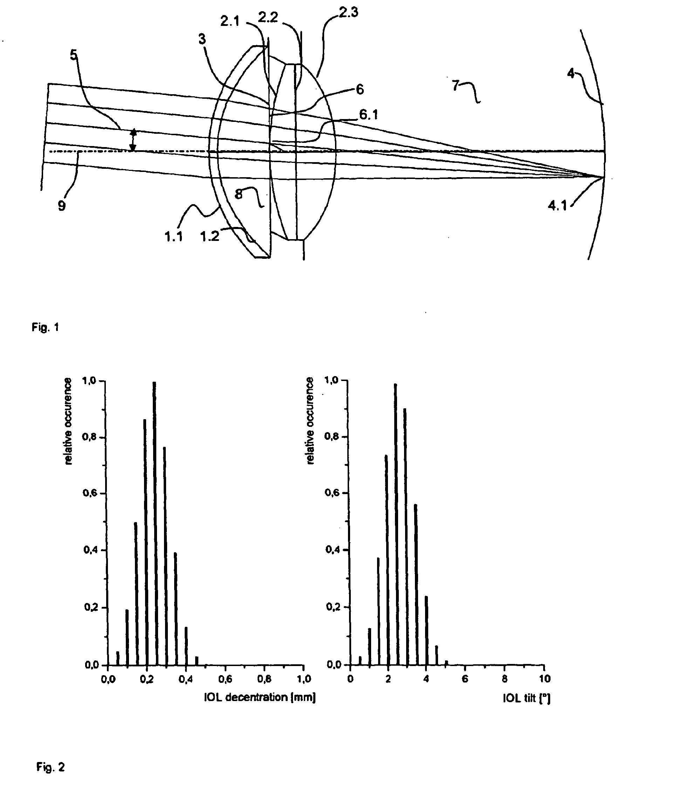

All the referred theoretical eye models, as well as the majority of published eye models rely on simplified ocular configurations of the human eye. The Cornea is reduced to a single surface element and the visual axis is assumed to match exactly the axis of symmetry of the eye. These reduced models try to mimic the optical system and aberrations of the human visual apparatus by use of single faced cornea models that apply some degree of asphericity in order to reflect the practically measurable performance. The authors proved that the referred eye models comply with measured results under given assumptions. However, these eye models disregard the specifics of the anatomy of human eye more or less systematic. The most comprehensive eye model currently available in literature was described by Liou and Brennan in the article "Anatomically accurate, finite model eye for optical modeling", J. Opt. Soc. Am. A/Vol. 14, No. 8/August 1997. The Liou-Brennan eye, as depicted in Fig. 1, represents the ocular anatomy very closely and preserves the optical properties and aberration characteristics of the human eye. This eye model comprises an aspherical Cornea with anterior 1.1 and posterior surface 2.2, as well as an aspherical gradient-index lens model. The anterior chamber is referred as 8, the vitreous body as 7, and the retina as 4. It takes into account that the visual axis 5 is tilted by 5° with respect to the axis of symmetry 9 of the eye to focus in the macular region 4.1 for the majority of the population. In addition the pupil 6 is slightly decentered by 0.5mm in nasal direction 6.1 for the majority of the population. The amount of spherical aberration (SA) is balanced by an aspheric cornea that introduces positive spherical aberration. An aspherical natural lens model, comprising two gradient index components with optical surfaces 2.1, 2.2, 2.3, provides negative SA to compensate for the corneal contribution. In total the optical train provides a little positive spherical aberration that is equivalent to measured data and helps to increase the depth of focus. In contrary to other model eyes is the Liou-Brennan eye consequentially not rotationally symmetric.

The design of the novel intra-ocular lenses uses an eye model that is based on the Liou-Brennan eye model as described by the surface listing:

| Surf | Comment | Radius | Thickness | Glass | Diameter | Conic |
|------|---------|--------|-----------|-------|----------|-------|
| OBJ | - | Infinity | Infinity | - | 0 | 0 |
| 1 | | Infinity | 1 | - | 4.495 | 0 |
| 2 | - | 0 | - | - | | |
| 3 | CORNEA FRONT | 7.77 | 0.5 | CORNEA LB | 12 | -0.18 |
| 4 | CORNEA BACK | 6.4 | 3.16 | WATER LB | 12 | -0.6 |
| 5 | PUPIL DEC 1 | - | 0 | - | - | |
| STO | PUPIL | Infinity | 0 | WATER LB | 4 | 0 |
| 7 | PUPIL DEC 2 | - | 0 | - | - | |
| 8 | LENS FRONT | 12.4 | 1.59 | - | 10 | -0.94 |
| 9 | LENS. CENTER | Infinity | 2.43 | - | 10 | 0 |
| 10 | LENS BACK | -8.1 | 16.27 | WATER LB | 10 | 0.96 |
| IMA | RETINA | -18 | | WATER LB | 20 | 0 |

The invention is based on specific geometry and/or shape modifications that apply to the anterior or posterior surface or both of novel intra-ocular lenses. The modified IOL surfaces comprise rotational symmetric deviations from a spherical shape. This procedure is commonly understood as aspherization of optical surfaces. Since aspherical surfaces are already well known from the prior art, the following sections will explain the novelties and enhancements and will furthermore explain the differentiation to common knowledge designs.

The new lens design is intended to improve the prior art in such a way that it provides measurable improvement of optical performance parameters that leads to patient perceivable improvement of visual acuity and contrast vision performance. In order to do so, the disclosed lens designs mimic the optical properties of the natural human lens under the conditions as described above in the Liou-Brennan eye model.

Substantial improvement of visual acuity is achieved by taking the statistics of potential lens misplacements into account for the lens design. The shape of the IOL surfaces is optimized to provide minimum sensitivity of the optical performance with regards to decentration and tilt of the implanted IOL. Different authors (Taketani at al: "Influence of intraocular lens optical design on higher-order aberrations", J. Cat. Refr. Surg, Vol31, May 2005) report a mean decentration of 0.1 mm - 0.25mm as the most likely case with ranges up to 1mm.

In addition the new IOL design fulfills the boundary condition of keeping the natural spherical aberration with the same amount than the human crystalline lens induces, for a broad range of pupil diameters. This allows the neuro-visual system to adapt quickly to the new implant, because the lifelong adaptation to the properties natural human doesn't need to be changed. Fig. 3 demonstrates that the new aspherical IOL approach provides the least deviation from the characteristics of the natural human eye (Liou-Brennan). It calculates the orthonormal Zemike coefficients, computed using the notation defined in R. Noll, "Zemike polynomials and atmospheric turbulence", J. Opt. Soc. Am., Vol. 66, No. 3, p207 (1976). This is also known as the "Born-Wolf-notation" (Born, Wolf "Principles of Optics", Chapter 1). The amount of this particular aberration coefficient is expressed in waves. [546nm]. The reference group consisting several IOLs of prior art (reference sign 30) shows significantly larger differences of SA at pupil zones up to 4.5mm (reference sign 31) and above.

An aspherical shape that allows the aforementioned optical performance and capabilities can be described by the equation:

$$z = \frac{cr^2}{1 + \sqrt{1 - (1+Q)c^2 r^2}} + k_2 r^2 + k_4 r^4 + k_6 r^6 + k_8 r^8 \qquad (1)$$

with

$$c = r_{curv}^{-1} \qquad \text{(curvature = 1 / base radius of curvature)}$$

r =  independent variable, radius about optical axis
Q =  conic constant
$k_n$ =  polynomial coefficient of order nRotational symmetric polynomial aspheric surfaces are described by a polynomial expansion of the deviation from a spherical (or aspheric described by a conic) surface. The even aspherical surface model uses only the even powers of the radial coordinate to describe the asphericity. The model uses the base radius of curvature and the conic constant.

The coefficients of the polynomial expansion, as well as the base radius are determined numerically in order to satisfy a least square fit to a particular merit function. This merit function accounts for the surgical statistics as described above and is minimized for optical performance. The merit function is represented by a set of different error and quality parameters that describe the desired optical performance. By definition the optimal state of the referred optical system is reached at a global minimum of the merit function. In order to optimize the IOL surface shape to achieve the advantageous properties as disclosed, the merit function is constructed using weighted wavefront aberration operands, weighted MTF operands, localized optical power operands as well as boundary constraints such as center thickness and edge thickness.

The following set of coefficients describes a new aspherical IOL at a base power of 220.

| surface | $r_{Curv}$ | Q | k2 | k4 | k6 | k8 |
|---|---|---|---|---|---|---|
| anterior | 7.1497 | 0.0 | 0.0 | 0.0 | 0.0 | 0.0 |
| posterior | -36.3903 | 0.0 | -6.8159E-003 | 1.0213E-003 | -6.2142E-005 | 0.0 |

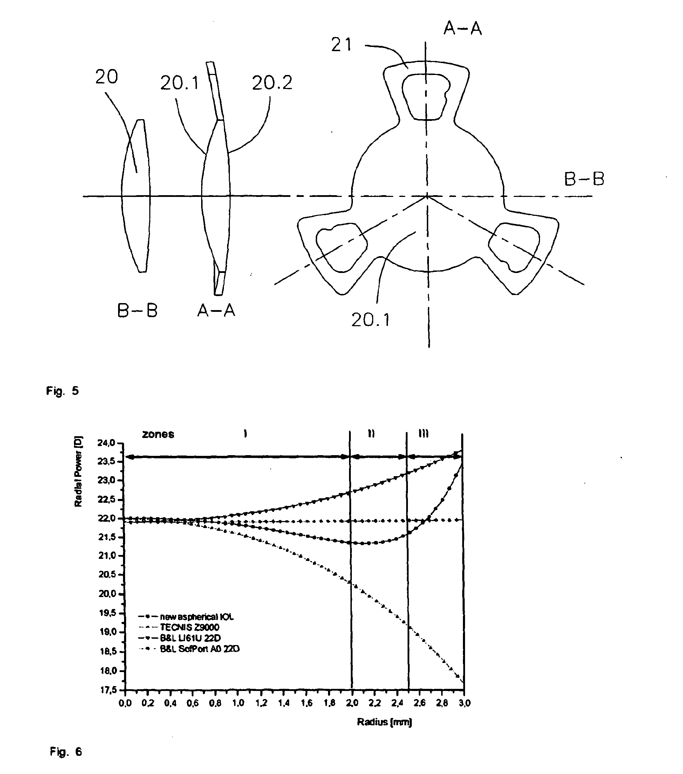

In accordance with formula (1) the required range of optical base powers can be easily calculated from 5D to 40D by setting the localized target power operands of the merit function to the desired power values and minimization of the remaining errors accordingly. An example of a possible layout can be seen in Fig. 4. The lens can be made of three pieces but is not limited to. Other preferred embodiments include 2-piece configurations or single piece IOLs as presented in Fig 5; 20 refers to the IOL body or the bulk material of the IOL; 21 is the haptics mechanism, 20.1 is the optical zone of the IOL. At least one of the optical surfaces 22.1 and 22.2 is made aspherical. With regards to the example as described above, surface 22.2 is set to be aspherical.

Fig. 6 shows the radial power profile of the modified IOL in comparison with other lens designs of prior art. The enhanced capabilities result from the particular characteristic of the radial power distribution as function of the radius normal to the optical axis. All IOLs start at their paraxial power of 22D (22 diopters) at a radius of 0mm. The power of the symmetric biconvex lens B&L LI61 increases continuously towards the lens edge. This indicates a significant amount of SA that exceeds the naturally given amount. In contrary the optical power of the TECNIS Z9000 decreases tremendously with increasing radius to provide negative SA that compensates for the corneal contributions. The drawback of this approach results from the high sensitivity of this design with regards to decentration of the IOL. The third example of prior art is the 'aberration free IOL' B&L SofPort A0. This lens claims independence of the optical performance with respect to decentration. This is accomplished be keeping the radial power at equal value to the paraxial power for all radii. In this case the lens is free from inherent SA. If this condition is fulfilled, decentration does not cause Coma errors that compromise image quality dramatically in the presence of decentration. Despite the referred advantages, this lens design suffers from another major drawback. The natural compensation effect of the human crystalline lens is fully disregarded. The image quality at the retina is therefore suboptimal for the patients since the full amount of corneal SA affects visual acuity in a negative way.

Fig. 6 teaches how the new IOL resolves the issues of the known IOL designs of the prior art. The distribution of the optical power vs. lens radius is selected in different zones in order to achieve optimal patient perceivable performance.

In zone I, the optical power is continuously and smoothly reduced in a pupil region starting from radius 0mm through 2.0mm. This pupil region is most active for photopic vision under bright vision conditions. The compensation for the corneal SA allows diffraction limited performance and improved contrast vision. In the zone II, a pupil region from r = 2.0mm through r = 2.5mm the optical power is less than the paraxial power in order to compensate for corneal SA at large pupils under mesopic conditions. The increase in optical power from r = 2.5 to r = 3mm in zone III reduces the sensitivity of the modulation transfer function with respect to decentration and tilt.

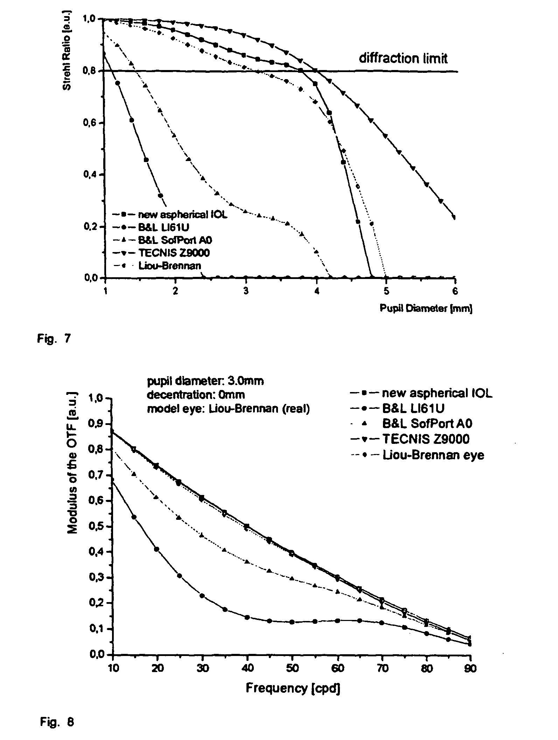

Fig. 7 shows, that the new lens design satisfies diffraction limited performance up to a pupil size of 4mm and equals the performance (Strehl-Ratio vs. Pupil diameter) of the natural crystalline lens for the entire pupil range.

Also the new lens design equals the diffraction limited optical performance (MTF) of the best prior art designs in case of physical pupil diameters of 3mm at no decentration (see Fig. 8).

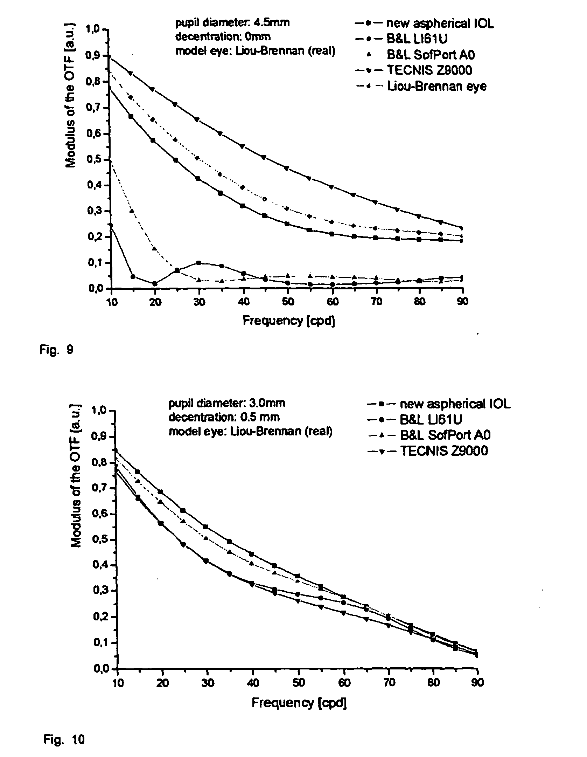

Fig. 9 shows that the new IOL equals the optical performance (MTF) of the natural human eye in case of physical pupil diameters up to 4.5 mm at no decentration.

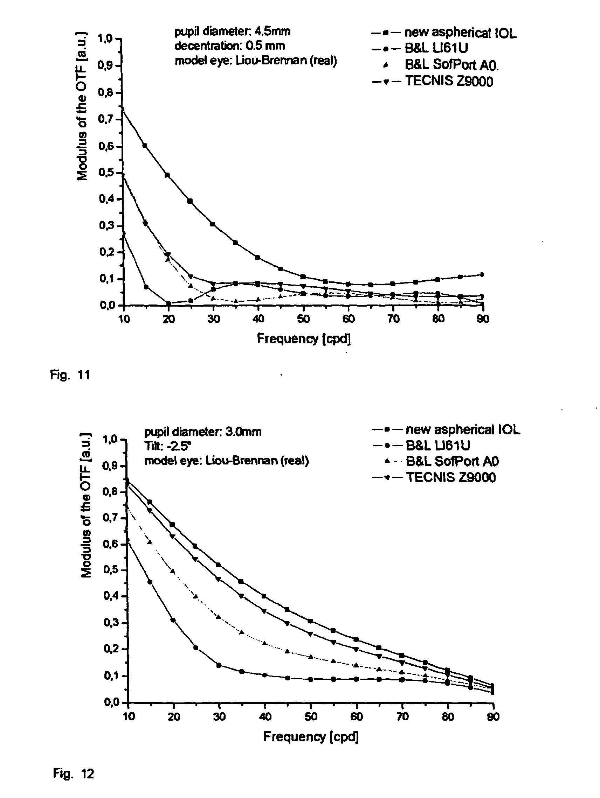

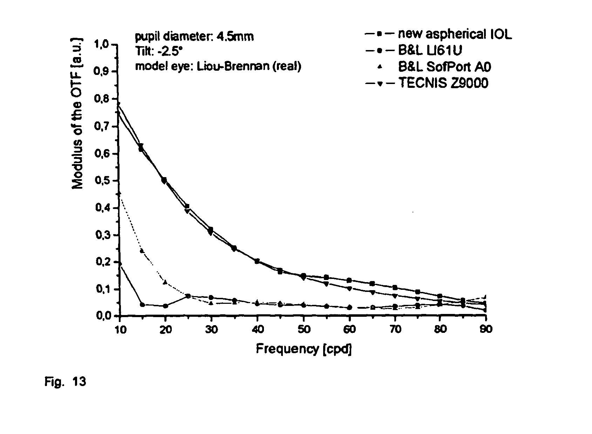

Fig. 10 and 11 show a significantly reduced sensitivity of the optical performance (MTF) with respect to decentration, while Fig. 12 and 13 show that the same is true with respect to tilt.

The foregoing description of the preferred embodiments of the invention have been presented for the purposes of illustration and description. It is not intended to be exhaustive or to limit the invention to the precise form disclosed. Many modifications and variations are possible in light of the above teaching.

**Claims**

1. Method for designing an intraocular lens capable of adjusting the aberrations of the eye in order to provide optimal vision correction to patients consisting of the following elements:

   • A mathematical model eye that describes the optical setup and performance of the natural human eye, comprising at least one aspherical corneal surface, a gradient index and / or aspherical crystalline lens model, a visual axis that is tilted with respect to the axis of symmetry of the eye and a decentered Iris that represents a decentered entrance pupil.
   • Determination of the performances of the mathematical model eye in terms of image quality and spherical aberrations in function of the pupil diameter.
   • Using a mathematical model that describes the statistics of potential lens misalignments and positioning errors induced by surgery or wound healing processes
   • Calculating the optical performance and resulting aberrations employing said mathematical eye model convoluted with the statistical model for lens displacements
   • And optical modeling an aspherical lens shape that replaces the natural human crystalline in the eye that provides optical power restoration while providing optical characteristics of the human lens in order to make the eye with the intraocular lens having aspherical shape to have the same amount of spherical aberrations and the same level of image quality than the mathematical model eye in function of the pupil diameter.

2. A method according to claim 1 wherein the radial distribution of refractive optical power is divided in at least three functional zones that account for photopic, mesopic, and scotopic vision.

3. A method according to claim 1 wherein modeling and optimization of the lens shape includes selecting the radii of base curvature of the anterior and posterior surfaces as well as the central thickness, the edge thickness and the refractive index.

4. A method according to claim 1 wherein the amount of spherical aberration of a mathematical model eye having the intraocular lens is maintained at the same level as the mathematical model eye having a human crystalline lens for pupil diameters ranging from greater 0 to 4 mm.

5. A method according to claim 1 wherein the modified lens shape is defined in terms of linear combination of polynomials.

6. A method according to claim 1 wherein the modified lens shape is defined by the equation:

$$z = \frac{cr^2}{1+\sqrt{1-(1+Q)c^2 r^2}} + k_2 r^2 + k_4 r^4 + k_6 r^6 + k_8 r^8$$

with

$c = r_{curv}^{-1}$ (Curvature = 1 / base radius of curvature)

r = independent variable, radius about optical axis
Q = conic constant
$k_n$ = polynomial coefficient of order n

7. A method according to claim 6 wherein $k_2$ is 0.

8. An aspheric intraocular lens designed by the method according to claim 1.

9. An aspheric intraocular lens according to claim 8, wherein the lens comprises an anterior and a posterior surface, whereby at least one of the anterior and posterior surface is aspheric and wherein the optical properties of these surfaces account for an spherical aberration equal or nearby the spherical aberration of the human eye.

**Patentansprüche**

1. Verfahren zum Entwerfen einer Intraokularlinse, die die Aberrationen des Auges ausgleichen kann, um Patienten eine optimale Sichtkorrektur zu liefern, bestehend aus den folgenden Elementen:

• ein mathematisches Modellauge, das den optischen Aufbau und die Leistung des natürlichen menschlichen Auges beschreibt, umfassend mindestens eine asphärische Hornhautoberfläche, ein Gradientenindex- und/ oder asphärisches kristallines Linsenmodell, eine Sichtachse, die bezüglich der Symmetrieachse des Auges geneigt ist, und eine dezentrierte Iris, die eine dezentrierte Eintrittspupille darstellt,
• Bestimmung der Leistung des mathematischen Modellauges hinsichtlich Bildqualität und sphärischen Aberrationen als Funktion des Pupillendurchmessers,
• Verwenden eines mathematischen Modells, das die Statistik potentieller Linsenfehlausrichtungen und Positionierungsfehler beschreibt, die durch Operations- oder Wundheilungsprozesse induziert werden,
• Berechnen der optimalen Leistung und resultierender Aberrationen unter Einsatz des mathematischen Augenmodells, gefaltet mit dem statistischen Modell für Linsenluxationen,
• und optisches Modellieren einer asphärischen Linsengestalt, die die natürliche menschliche Kristalllinse in dem Auge ersetzt, das eine Brechwertwiederherstellung bereitstellt und dabei optische Charakteristika der menschlichen Linse bereitstellt, um zu bewirken, dass das Auge mit der Intraokularlinse mit asphärischer Gestalt das gleiche Ausmaß an sphärischen Aberrationen und die gleiche Höhe der Bildqualität wie das mathematische Modellauge als Funktion des Pupillendurchmessers aufweist.

2. Verfahren nach Anspruch 1, wobei die radiale Verteilung der Brechleistung in mindestens drei funktionale Zonen unterteilt wird, die photopisches, mesopisches und skotopisches Sehen ausmachen.

3. Verfahren nach Anspruch 1, wobei Modellierung und Optimierung der Linsengestalt das Wählen von Basiskrümmungsradien der vorderen und hinteren Oberfläche sowie der zentralen Dicke, der Randdicke und des Brechungsindexes beinhaltet.

4. Verfahren nach Anspruch 1, wobei das Ausmaß an sphärischer Aberration eines mathematischen Modellauges mit der Intraokularlinse auf der gleichen Höhe gehalten wird wie das mathematische Modellauge mit einer menschlichen Kristalllinse für Pupillendurchmesser im Bereich von größer als 0 bis 4 mm.

**5.** Verfahren nach Anspruch 1, wobei die modifizierte Linsengestalt hinsichtlich einer linearen Kombination von Polynomen definiert wird.

**6.** Verfahren nach Anspruch 1, wobei die modifizierte Linsengestalt durch die folgende Gleichung definiert ist:

$$z = \frac{cr^2}{1+\sqrt{1-(1+Q)c^2r^2}} + k_2 r^2 + k_4 r^4 + k_6 r^6 + k_8 r^8$$

wobei

$c = r_{cur}^{-1}$ (Krümmung = 1/Basiskrümmungsradius)

r = unabhängige Variable, Radius um optische Achse
Q = konische Konstante
$k_n$ = Polynomkoeffizient der Größenordnung n.

**7.** Verfahren nach Anspruch 6, wobei $k_2$ 0 ist.

**8.** Asphärische Intraokularlinse, durch das Verfahren nach Anspruch 1 ausgelegt.

**9.** Asphärische Intraokularlinse nach Anspruch 8, wobei die Linse eine vordere und eine hintere Oberfläche umfasst, wobei mindestens eine der vorderen und hinteren Oberfläche asphärisch ist und wobei die optischen Eigenschaften dieser Oberflächen eine sphärische Aberration berücksichtigen, die gleich der sphärischen Aberration des menschlichen Auges oder nahe dieser liegt.

**Revendications**

**1.** Procédé pour la conception d'une lentille intraoculaire capable d'ajuster les aberrations de l'oeil afin de procurer à des patients une correction de vision optimale, comprenant les éléments suivants :

    - un modèle mathématique d'oeil qui décrit la configuration optique et les performances de l'oeil humain naturel, comprenant au moins une surface cornéenne asphérique, un gradient d'indice et/ou un modèle de cristallin asphérique, un axe visuel qui est incliné par rapport à l'axe de symétrie de l'oeil et un iris décentré qui représente une pupille d'entrée décentrée ;
    - la détermination des performances du modèle mathématique de l'oeil en ce qui concerne la qualité d'image et les aberrations sphériques en fonction du diamètre de pupille ;
    - l'utilisation d'un modèle mathématique qui décrit les statistiques des désalignements potentiels de la lentille et les erreurs de positionnement induites par la chirurgie ou par les processus de cicatrisation ;
    - le calcul des performances optiques et des aberrations qui en résultent en utilisant ledit modèle mathématique de l'oeil convolué avec le modèle statistique des déplacements de lentille ;
    - et la modélisation optique d'une forme de lentille asphérique qui remplace le cristallin naturel humain dans l'oeil et permet la restauration de la puissance optique tout en offrant des caractéristiques optiques du cristallin humain afin de faire en sorte que l'oeil muni de la lentille intraoculaire de forme asphérique ait la même quantité d'aberrations sphériques et le même niveau de qualité d'image que le modèle mathématique d'oeil en fonction du diamètre de pupille.

**2.** Procédé selon la revendication 1, dans lequel la répartition radiale de la puissance optique de réfraction est divisée en au moins trois zones fonctionnelles qui sont responsables de la vision photopique, mésopique et scotopique.

**3.** Procédé selon la revendication 1, dans lequel la modélisation et l'optimisation de la forme de lentille comprennent le choix des rayons de courbure de base des surfaces antérieure et postérieure, ainsi que l'épaisseur centrale, l'épaisseur de bord et l'indice de réfraction.

**4.** Procédé selon la revendication 1, dans lequel la quantité d'aberration sphérique d'un modèle mathématique d'oeil de la lentille intraoculaire est maintenue au même niveau que celui du modèle mathématique d'oeil comportant un cristallin humain pour des diamètres de pupille allant de plus de 0 à 4 mm.

5. Procédé selon la revendication 1, dans lequel la forme de lentille modifiée est définie par une combinaison linéaire de polynômes.

6. Procédé selon la revendication 1, dans lequel la forme de lentille modifiée est définie par l'équation :

$$z = \frac{cr^2}{1+\sqrt{1-(1+Q)c^2r^2}} + k_2 r^2 + k_4 r^4 + k_6 r^6 + k_8 r^8$$

avec

$$c = r_{courb}^{-1}$$  (Courbure = 1 / rayon de courbure de base)

r = variable indépendante, rayon par rapport à l'axe optique
Q = constante de conicité
$K_n$ = coefficient polynomial d'ordre n.

7. Procédé selon la revendication 6, dans lequel $k_2$ est égal à 0.

8. Lentille intraoculaire asphérique conçue au moyen du procédé selon la revendication 1.

9. Lentille intraoculaire asphérique selon la revendication 8, dans laquelle la lentille comprend des surfaces antérieure et postérieure, au moins l'une des surfaces antérieure et postérieure étant asphérique, et dans laquelle les propriétés optiques de ces surfaces sont responsables d'une aberration sphérique égale à, ou à proche de l'aberration sphérique de l'oeil humain.

Fig. 1

Fig. 2

12

Fig. 3

Fig. 4

Fig. 5

Fig. 6

Fig. 7

Fig. 8

Fig. 9

Fig. 10

pupil diameter: 4.5mm
decentration: 0.5 mm
model eye: Liou-Brennan (real)

—■— new aspherical IOL
—●— B&L LI61U
▲ B&L SofPort AO.
—▼— TECNIS Z9000

Fig. 11

pupil diameter: 3.0mm
Tilt: -2.5°
model eye: Liou-Brennan (real)

—■— new aspherical IOL
—●— B&L LI61U
▲-- B&L SofPort AO
—▼— TECNIS Z9000

Fig. 12

Fig. 13

18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6609793 B2
- US 2005203619 A1
- WO 2004090611 A3
- US 20040230299 A
- WO 2005046527 A
- US 6126286 A
- EP 1402852 A
- EP 1424049 A
- EP 1003446 A

**Non-patent literature cited in the description**

- **Liou ; Brennan.** Anatomically accurate, finite model eye for optical modeling. *J. Opt. Soc. Am. A,* August 1997, vol. 14 (8
- **Taketani.** Influence of intraocular lens optical design on higher-order aberrations. *J. Cat. Refr. Surg,* May 2005, vol. 31
- **R. Noll.** Zemike polynomials and atmospheric turbulence. *J. Opt. Soc. Am.,* 1976, vol. 66 (3), 207
- Born-Wolf-notation. **Born ; Wolf.** Principles of Optics